**Europäisches Patentamt**

**European Patent Office**

**Office européen des brevets**

(19)

(11) Veröffentlichungsnummer: **0 124 004**
**A2**

(12)

# EUROPÄISCHE PATENTANMELDUNG

(21) Anmeldenummer: **84104308.6**

(22) Anmeldetag: **16.04.84**

(51) Int. Cl.³: **C 12 Q 1/44**

(30) Priorität: **15.04.83  DE 3313647**

(43) Veröffentlichungstag der Anmeldung: **07.11.84**
**Patentblatt 84/45**

(84) Benannte Vertragsstaaten: **AT BE CH FR GB IT LI NL**

(71) Anmelder: **Schöbel, Barbara, Dr., Winzerweg 2,
D-6220 Rüdesheim/Rhein (DE)**

(72) Erfinder: **Schöbel, Barbara, Dr., Winzerweg 2,
D-6220 Rüdesheim/Rhein (DE)**

(74) Vertreter: **Dipl.-Phys.Dr. Manitz Dipl.-Ing. Finsterwald
Dipl.-Ing. Grämkow Dipl.-Chem.Dr. Heyn
Dipl.-Phys.Rotermund, B.Sc. Morgan
Robert-Koch-Strasse 1, D-8000 München 22 (DE)**

(54) **Verfahren zur Bestimmung von Pankreas-Lipase.**

(57) Ein Verfahren zur Bestimmung von Pankreas-Lipase zeichnet sich dadurch aus, daß als Substrat Mono- oder Diester des Dihydroxiacetons mit gesättigten und/oder ungesättigten Karbonsäuren einer Kettenlänge von vorzugsweise 8 bis 20 Kohlenstoffatomen verwendet wird.

0124004

Verfahren zur Bestimmung von Pankreas-Lipase

---

Gegenstand der Erfindung ist ein Verfahren zur Bestimmung von Pankreas-Lipase (EC 3.1.1.3) d a d u r c h g e k e n n - z e i c h n e t , daß als Substrat Mono- oder Diester des Dihydroxiacetons verwendet werden.

Die Messung der Pankreas-Lipase spielt in der medizinischen Diagnostik eine bedeutende Rolle. Hierbei kommt es darauf an, spezifisch die Pankreas-Lipase neben den unspezifischen Esterasen (EC 3.1.1.1) in den biologischen Proben quantitativ zu bestimmen. Natürliche Substrate für die Pankreas-Lipase sind die Tri- und Diglyceride, Ester des Glycerins mit langkettigen Carbonsäuren. Für die Messung der Pankreas-Lipase werden daher in der Regel Olivenölsuspensionen verwendet. Durch die Lipasespaltung der Ester werden die Fetttröpfchen verseift und der Trübungsgrad nimmt ab. Durch turbidimetrische Messungen kann so indirekt auf eine vorhandene Lipase-Aktivität geschlossen werden. Problematisch bei dieser Bestimmungsmethode ist die Standardisierung der Fettsuspension (-emulsion). Darüberhinaus hängt der Grad der Lichtstreuung stark vom Verteilungsspektrum der Teilchengrößen ab.

Synthetische Substrate wie der 8-Phenyloctansäurevinylester unterscheiden sich strukturell erheblich vom natürlichen Substrat. Darüberhinaus wird der bei dieser Methode entstehende Acetaldehyd über NADH im UV-Bereich gemessen. Das bedeutet relativ geringe Nachweisempfindlichkeit bei hoher Störanfälligkeit durch Kolloide und Probenbegleitstoffe.

Überraschenderweise wurde nun festgestellt, daß die erfindungsgemäßen Substanzen gute Substrate für die Pankreas-Lipase darstellen. Sie werden rasch durch die Pankreas-Lipase zu Dihydroxiaceton und Fettsäuren hydrolysiert. Die unspezifischen Esterasen (EC 3.1.1.1) zeigen hingegen praktisch keine Spaltung der Substanzen.

Bei den erfindungsgemäßen Substanzen handelt es sich somit um wohldefinierte und gleichzeitig spezifische Substrate für die Pankreas-Lipase.

Das bei der Hydrolyse entstehende Dihydroxiaceton kann mit Hilfe von Galaktose-Oxidase in bekannter Weise unter Bildung von Wasserstoffperoxid oxidiert werden. Dieses wird mit einer der beschriebenen Methoden sodann quantitativ gemessen.

Das erfindungsgemäße Verfahren ist in folgenden Beispielen näher erläutert:

Beispiel 1: 20 µl des zu untersuchenden Serums werden zu 1,0 ml der folgenden Reaktionslösung gegeben - Tris/HCl-Puffer 50mmol/l pH 7,0; 0,15 mol/l NaCl; 1 mmol/l $CaCl_2$; 5 mmol/l Na-taurodesoxicholat; 1000 U/l Galaktose-Oxidase; 2500 U/l Peroxidase; 50 mmol/l Dihydroxiacetondipalmitinsäureester plus 10 µl Wursters Reagenz (10 mmol/l).Es wird bei 37°C die Extinktionszunahme bei 610 nm im Abstand von je einer Minute bestimmt und per Faktor die Lipaseaktivität in U/l berechnet.

Beispiel 2: Analoger Versuch wie unter Beispiel 1, aber mit Zusatz von 2 mg/l Colipase und 20 mmol/l Desoxicholat anstelle des Na-taurodesoxicholates. Messung der Extinktionszunahme bei Hg 623 Filter im Abstand von jeweils einer Minute.Als Substrat wurde hier Dihydroxiacetonmonolinolsäureester (90 mmol/l) verwendet.

0124004

Beispiel 3: Analoger Versuch wie unter Beispiel 1, aber als Substrat 85 mmol/l Dihydroxiacetondioctansäureester. Ablesung der Extinktionszunahme alle 2 Minuten bei 562 nm. Eichung der Methode mit hochreiner Schweine-Pankreas-Lipase.

Beispiel 4: Analoger Versuch wie unter Beispiel 1, aber unter Zusatz von 0,1 mmol/l Phenylmethylsulfonylfluorid. Anstelle des Dihydroxiacetondipalmitinsäureesters wurde hier als Substrat Dihydroxiacetondi-(8-Äthyldecansäure)-ester (80 mmol/l) verwendet. Die Messung der Extinktionszunahme erfolgte alle 2 Minuten bei 610 nm.

Patentansprüche

1. Verfahren zur Bestimmung von Pankreas-Lipase ,
d a d u r c h  g e k e n n z e i c h n e t , daß als
Substrat Mono- oder Diester des Dihydroxiacetons mit
gesättigten und/oder ungesättigten Carbonsäuren einer
Kettenlänge von vorzugsweise 8 bis 20 Kohlenstoffatomen
verwendet wird.

2. Verfahren nach Anspruch 1, d a d u r c h  g e k e n n-
z e i c h n e t , daß das erfindungsgemäße Substrat
zusammen mit den Pankreas-Lipase Aktivatoren Na-taurodesoxicholat, Desoxicholat und/oder Colipase verwendet
wird.

3. Verfahren nach Anspruch 1, d a d u r c h  g e k e n n-
z e i c h n e t , daß das erfindungsgemäße Substrat
zusammen mit Hemmern der unspezifischen Esterasen,Diisopropylfluorophosphat oder Phenylmethylsulfonylfluorid,verwendet wird.

4. Verfahren nach Anspruch 1, d a d u r c h  g e k e n n-
z e i c h n e t , daß das bei der Pankreas-Lipaseeinwirkung aus dem erfindungsgemäßen Substrat gebildete
Dihydroxiaceton mittels Galaktose-Oxidase unter Bildung
von meßbarem Wasserstoffperoxid oxidiert wird.

5. Reagenz zur Bestimmung von Pankreas-Lipase , d a -
d u r c h  g e k e n n z e i c h n e t , daß es als
Substrat die erfindungsgemäßen Mono- oder Diester des
Dihydroxiacetons enthält.